# EUROPEAN PATENT SPECIFICATION

(11) **EP 1 840 026 B1**
(45) Date of publication and mention of the grant of the patent: **10.07.2019**
(21) Application number: 07105301.1
(22) Date of filing: 30.03.2007
(51) Int. Cl.: B63H 1/16, B63H 21/17

(54) **PRODUCT TRANSPORT SYSTEM USING AN ARCHIMEDEAN SCREW**
PRODUKTTRASNPORTSYSTEM MIT EINER ARCHIMEDISCHEN SCHRAUBE
SYSTÈME DE TRANSPORT DE PRODUITS UTILISANT UNE VIS D'ARCHIMÈDE

(30) Priority: 31.03.2006 IT MI20060633
(43) Date of publication of application: 03.10.2007
(73) Proprietor: GORIZIANE GROUP S.p.A., 34070 Villese (GO) (IT)
(72) Inventor: Novelli, Flavio, 34077 Ronchi dei Legionari (Gorizia) (IT)
(74) Representative: Bonatto, Marco

(56) References cited:
- EP-A2- 0 786 402
- EP-A2- 1 120 570
- EP-A2- 1 122 165
- FR-A1- 2 825 679
- FR-A1- 2 839 266
- JP-A- S60 106 709
- US-A- 5 181 868
- US-A- 5 209 650
- US-B1- 6 394 769

## Description

The present invention concerns a product transportation system that can be used for example as a conveyor belt for products.
For the aforementioned application in the prior art the rotation of the Archimedean screw takes place through a central axis onto which the Archimedean screw is fixedly attached, to which the necessary power is applied through an external motor.

The presence of a central shaft and of the external motor can often be a hindrance and they can prevent a continuous flow of the product without an inevitable deviation of the transported product before it has reached the motor system.
The Applicant has set itself the problem of supporting the traction of an Archimedean screw so that the total bulk of the product transport system as a whole is reduced whilst maintaining the same power that can be dispensed and transferred to the rotation of the Archimedean screw.

The Applicant has made a product transportation system using an Archimedean screw in which the movement is transferred to the Archimedean screw by a containment cylinder that supports the Archimedean screw and that rotates with it.
This choice is very important because it allows the central axis to be reduced the peripheral traction allows water, or any other transportable product, to be conveyed, without the hindrance of the motor that prevents the continuous flow without an inevitable deviation of the transported product before the motor system is reached.

An aspect of the present invention concerns a product transportation system having the features according to claim **1.**
The documents US5181868 and EP1122165A1 disclose propulsion assemblies having some of the features of claim **1.**
The document JPS60-106709 discloses a conveyor for powder and granular material, provided with an Archimedean screw. Unlike the product transportation system according to the present invention, such conveyor lacks a support cylinder, a cylindrical rotor and a cylindrical stator of an electric motor.
Furthermore document JPS60-106709 does not disclose the following features of claim 1:
- a motor cylindrical rotor fixedly attached onto the outer surface of the Archimedean screw group;
- an Archimedean screw extending radially from the support cylinder up to said central shaft;
- a protective tube associated with the support cylinder of the Archimedean screw group inside which the traction system using the Archimedean screw rotates and inside which the products are transported.

Further purposes and advantages of the system according to the present invention shall become clearer from the following description and from the attached drawings, provided purely as a non-limiting example, in which:
- figures **1**a and **1**b illustrate a view of the Archimedean screw of the rotor of the system according to the present invention;
- figures **2**a and **2**b illustrate a view of the Archimedean screw of the rotor and of the stator assembled together according to the present invention;
- figure **3** illustrates a view of the traction system using an Archimedean screw used as traction for a seagoing vessel (not claimed);
- figure **4** illustrates a view of the traction system using an Archimedean screw used as a pump for fluids (not claimed);
- figure **5** illustrates a view of the traction system using an Archimedean screw used as a system for transporting products according to the invention;
- figure **6** illustrates a view of the traction system using an Archimedean screw used as a heart pump (not claimed).

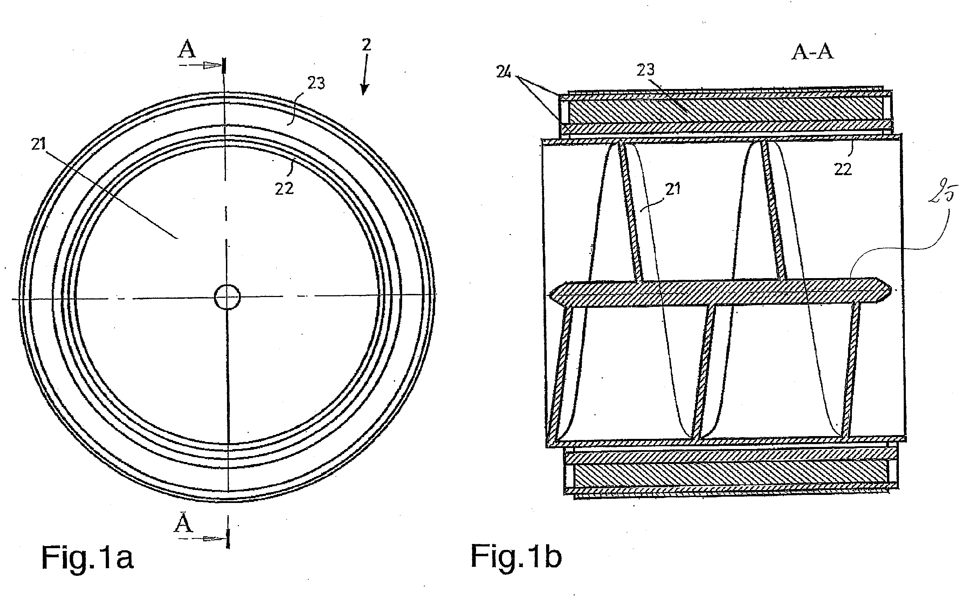

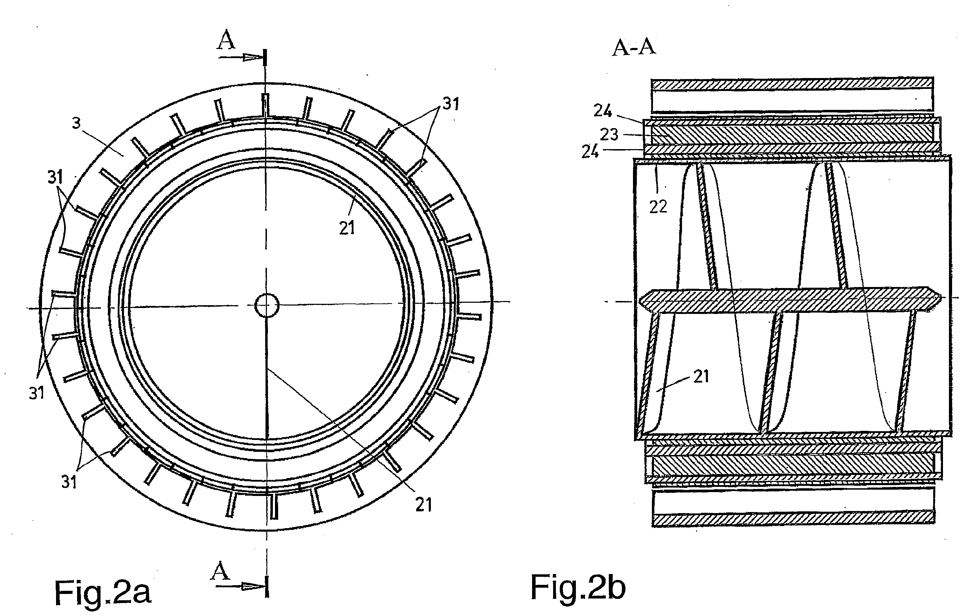

With reference to the quoted figures, a product transportation system using an Archimedean screw is made through substantially two parts that can be assembled together. In particular, the system comprises an Archimedean screw group **2,** illustrated in particular in figure **1****,** formed from an Archimedean screw **21** associated, for example welded, on a support cylinder **22** on the outer surface of which a cylindrical rotor **23** of an electric motor, for example of the asynchronous type, is fixedly attached; on the rotor **23** there is the characteristic cage **24.** Figure **2** illustrates the system as a whole in which the Archimedean screw/rotor group is associated with a cylindrical stator **3,** fitted onto said rotor, having recesses for the windings **31,** obtaining the complete motor.

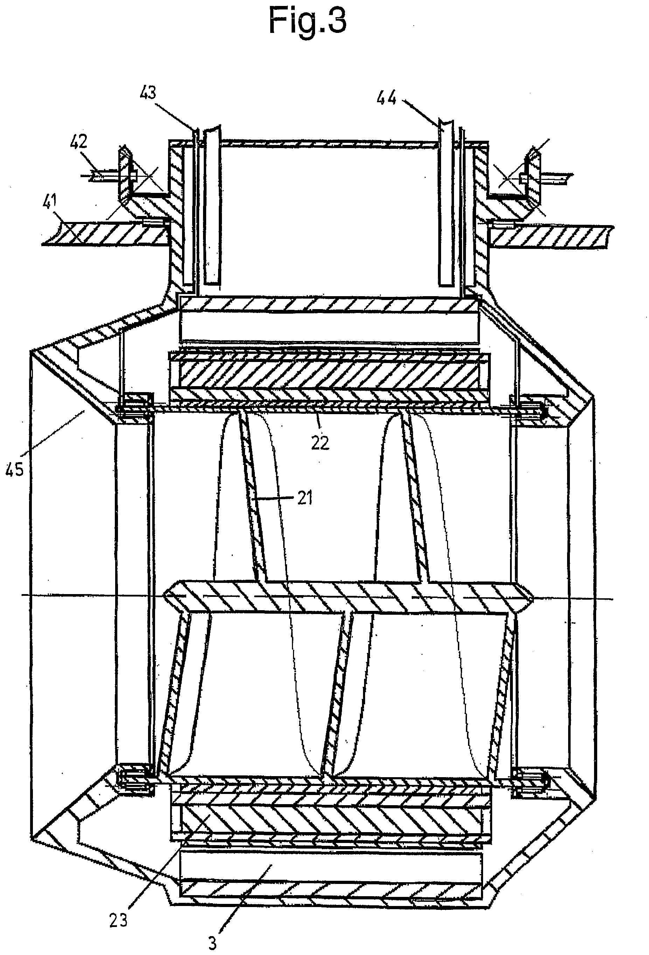

Figure **3** illustrates the system housed in its seat on the deck of a ship **41** (not claimed) and it is rotated in its seat preferably through a geared rotation system **42;** moreover, the figure highlights the ducts for the lubrication of the bearings **43,** the ducts for the electrical cables **44,** a conveyor of the flow of water **45** towards the Archimedean screw **21,** the rotor **23** and the stator **3.**

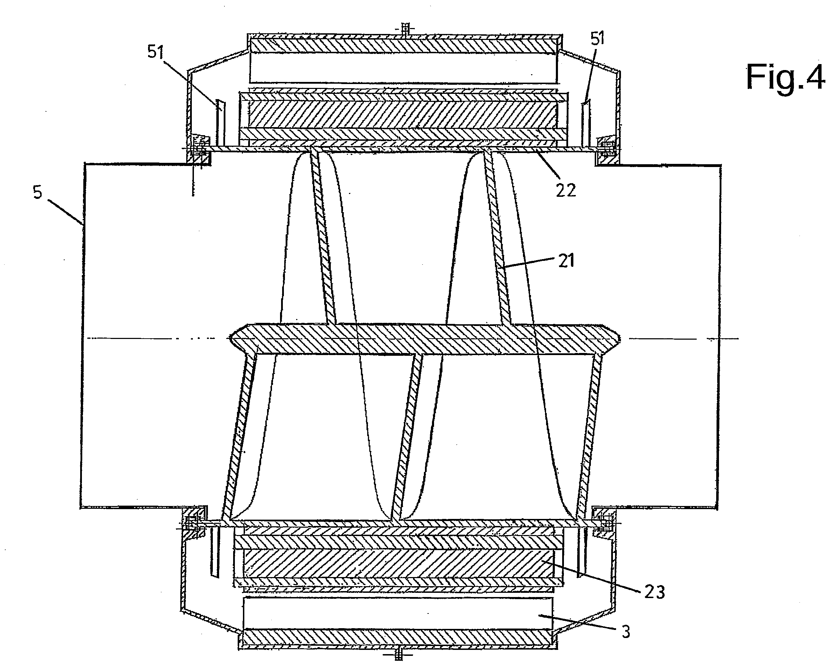

Figure **4** represents the system in a configuration suitable for operating as a simple pump for liquids (not claimed) having a fitting tube **5** with an external circuit that is associated with the support cylinder **22** of the Archimedean screw group.
In this case the fitting can be used with an outer strap that locks the flexible tube (not represented) fitted onto the fitting tube going in and out. Moreover, by equipping the fitting tube with a gas-type threading it is possible to obtain the coupling with the circuit outside of the pump.

The pump is also provided with cooling and ventilation flaps **51.** The power and the overall size of the pump are very small compared to the system for ship traction; for example, the diameter of the fitting tube could be two or three inches with a minimum power of **150-200** Watts.

A transport system using an Archimedean screw according to the present invention can be used instead of conveyor belts, since compared to them it has substantial advantages: indeed, with the system using an Archimedean screw it is possible to transfer the products without the wind or external agents being able to alter the transported product; moreover, it is possible to substantially vary the transportation angle, to vary and precisely evaluate the amount of material transported even in different weather conditions.

With the system using an Archimedean screw both liquids and loose solids of small unitary size, such as sand, salt, flour and grain, can be transported.
The system according to the present invention fixes the Archimedean screw to the containment cylinder and, on this, the rotor of the electric motor is fixed. In this way, the liquid and the loose solids transported do not meet any obstacle at the end of the Archimedean screw, which can be lengthened or shortened as desired with another Archimedean screw since there is no hindrance to connection; moreover, it is possible to make a very long Archimedean screw since many motors can be positioned circumferentially, until the necessary power is reached.

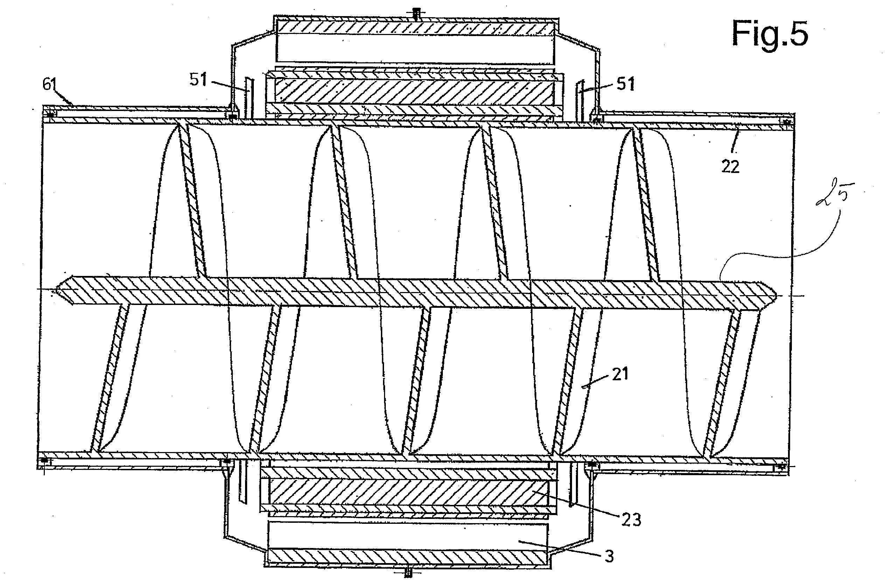

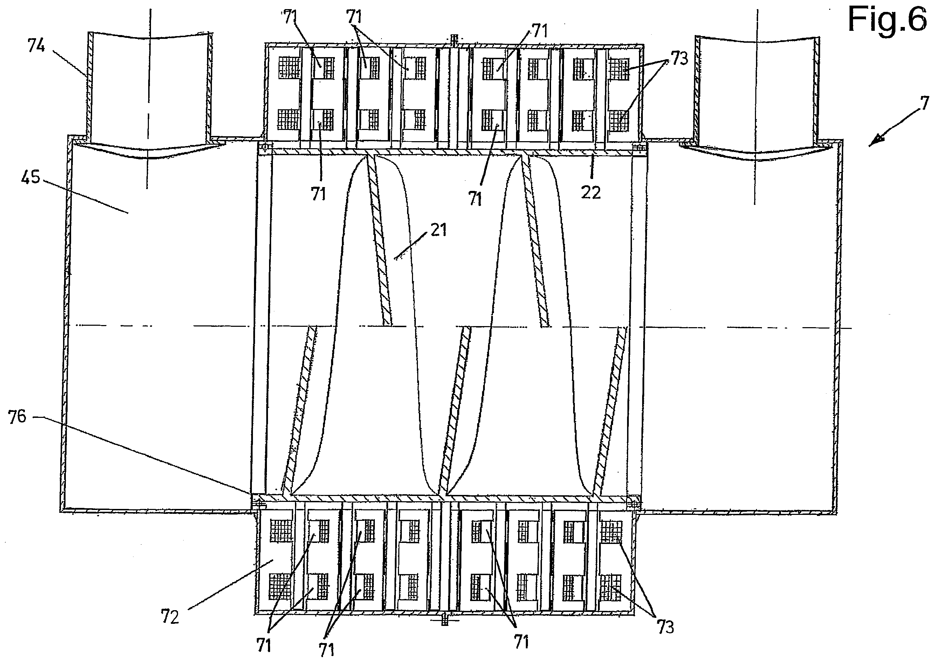

Figure **5** represents such a product transportation system made through the traction system using an Archimedean screw according to the present invention having a protective tube **61,** which is associated with the support cylinder **22** of the Archimedean screw group inside which the traction system using an Archimedean screw rotates and inside which the products are transported.
A further application of the transport system using an Archimedean screw is that relating to making a heart pump **7,** illustrated in figure **6****.** In particular, this type of pump has the task of supplementing the work of the heart; the pump is arranged in series with the weakened heart and supplements its blood pumping functions.

The electric motor is of the transverse flux type having permanent magnets **71,** ferromagnetic cores **72** and electrical windings **73.** The electrical power supply to the motor can be obtained through an induction system that allows the energy to be received through a subcutaneous coil; in this way, one avoids passing conductors through the skin and the consequent possibility of infection.
The power necessary in this application is very small but to make it easier to absorb the heat produced by the Joule effect the excitation circuits of the stator are fractioned and the small magnets must be the maximum number possible, so as to be able to use high frequencies.

The haematic flow goes into the pump through an inlet tube **74** that reaches a tank **75** that is associated with the support cylinder **22,** and then through the Archimedean screw **21** the liquid is thrusted and accelerated towards the outlet on the opposite side of the support cylinder; the flow is protected, in the passage from the tank to the Archimedean screw, by a joint cover **76.**
This type of pump is particularly useful since it reduces to the minimum the trauma suffered by the haematic liquid in its acceleration by varying the pitch of the Archimedean screw and carrying out a slow but continuous acceleration, until the necessary outlet pressure and speed is reached.

## Claims

1. Product transportation system comprising an Archimedean screw group (**2**) formed from an Archimedean screw (**21**) associated with a support cylinder (**22**), a cylindrical rotor (**23**) of an electric motor fixedly attached onto the outer surface of the Archimedean screw group and a cylindrical stator (**3**) fitted onto the outside of said rotor, wherein the Archimedean screw (**21**) further comprises a central shaft (**25**) arranged along the central rotation axis of the Archimedean screw (**21**) and of the support cylinder (**22**), and the Archimedean screw (**21**) extends radially from the support cylinder (**22**) up to said central shaft and wherein said product transportation system further comprises a protective tube (**61**) associated with the support cylinder (**22**) of the Archimedean screw group inside which the traction system using the Archimedean screw rotates and inside which the products are transported.

2. System according to claim **1,** wherein such a motor is an asynchronous electric motor having cages (**24**) on the rotor (**23**) and windings (**31**) on the stator (**3**).

## Patentansprüche

1. Produkttransportsystem, umfassend eine archimedische Schraubengruppe (2), die gebildet ist aus einer mit einem Trägerzylinder (22) verbundenen archimedischen Schraube (21), einem zylindrischen Rotor (23) eines fest an der Außenfläche der archimedischen Schraubengruppe angeordneten Elektromotor und einem an der Außenseite des Rotors eingebauten zylindrischen Stator (3), wobei die archimedische Schraube (21) ferner einen zentrale Welle (25) umfasst, die auf der zentralen Drehachse der archimedischen Schraube (21) und des Trägerzylinders (22) angeordnet ist, und die archimedische Schraube (21) sich radial aus dem Trägerzylinder (22) bis zu der zentralen Welle erstreckt und wobei das Produkttransportsystem ferner ein Schutzrohr (61) umfasst, das mit dem Trägerzylinder (22) der archimedischen Schraubengruppe verbunden ist, in welchem das die archimedische Schraube verwendende Antriebssystem sich dreht und in welchem die Produkte transportiert werden.

2. System nach Anspruch 1, wobei ein derartiger Motor ein asynchroner Elektromotor mit Käfigen (24) auf dem Rotor (23) und Wicklungen (31) auf dem Stator (3) ist.

## Revendications

1. Système de transport de produits comprenant un groupe de vis d'Archimède (**2**) formé d'une vis d'Archimède (**21**) associée à un cylindre de support (**22**), un rotor cylindrique (**23**) d'un moteur électrique fixé fermement sur la surface extérieure du groupe de vis d'Archimède et un stator cylindrique (**3**) monté sur l'extérieur dudit rotor, dans lequel la vis d'Archimède (**21**) comprend en outre un arbre central (**25**) disposé le long de l'axe de rotation central de la vis d'Archimède (**21**) et du cylindre de support (**22**), et la vis d'Archimède (**21**) s'étend radialement du cylindre de support (**22**) jusqu'audit arbre central et dans lequel ledit système de transport de produits comprend en outre un tube protecteur (**61**) associé au cylindre de support (**22**) du groupe de vis d'Archimède à l'intérieur duquel tourne le système de traction utilisant la vis d'Archimède et à l'intérieur duquel les produits sont transportés.

2. Système selon la revendication **1,** dans lequel un tel moteur est un moteur électrique asynchrone ayant des cages (**24**) sur le rotor (**23**) et des enroulements (**31**) sur le stator (**3**).
